(19) Europäisches Patentamt
European Patent Office
Office européen des brevets

(11) EP 1 674 453 A1

(12) EUROPÄISCHE PATENTANMELDUNG

(43) Veröffentlichungstag:
**28.06.2006 Patentblatt 2006/26**

(51) Int Cl.:
***C07C 319/04*** *(2006.01)* ***C07C 321/04*** *(2006.01)*

(21) Anmeldenummer: **05027316.8**

(22) Anmeldetag: **14.12.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **15.12.2004 DE 102004060320**

(71) Anmelder: **BASF Aktiengesellschaft**
**67056 Ludwigshafen (DE)**

(72) Erfinder:
- **Müller, Christian**
  **68167 Mannheim (DE)**
- **Weber, Markus, Dr.**
  **67061 Ludwigshafen (DE)**
- **Stroefer, Eckhard, Dr.**
  **68163 Mannheim (DE)**
- **Wloka, Veronika, Dr.**
  **68163 Mannheim (DE)**
- **Faderl, Jürgen, Dr.**
  **68549 Ilvesheim (DE)**

(74) Vertreter: **Isenbruck, Günter**
**Isenbruck, Bösl, Hörschler, Wichmann, Huhn**
**Patentanwälte**
**Theodor-Heuss-Anlage 12**
**68165 Mannheim (DE)**

# (54) Verfahren zur Herstellung von Thiolen

(57) Die vorliegende Erfindung betrifft ein Verfahren zur Herstellung von Alkyl-Thiolen durch Addition von Schwefelwasserstoff an Doppelbindungen von $C_6$-$C_{20}$-Olefinen bei einer Temperatur von 20 bis 150 °C und einem Druck von 1 bis 40 bar, wobei die Reaktion in Gegenwart mindestens einer organischen, flüssigen Säure durchgeführt wird, sowie die Verwendung von organischen, flüssigen Säuren als Katalysator zur Erhöhung der Selektivität und/oder Reaktionsgeschwindigkeit bei der Addition von Schwefelwasserstoff an Doppelbindungen von $C_6$-$C_{20}$-Olefinen zur Herstellung von Alkyl-Thiolen.

EP 1 674 453 A1

**Beschreibung**

Die vorliegende Erfindung betrifft ein Verfahren zur Herstellung von Alkyl-Thiolen durch Addition von Schwefelwasserstoff an die Doppelbindungen von Olefinen.

Alkylthiole mit 10 bis 30 Kohlenstoffatomen sind bekannte Verbindungen. Alkylthiole oder Gemische dieser Verbindungen werden üblicherweise durch sauer katalysierte elektrophile Addition von Schwefelwasserstoff ($H_2S$) an Olefine erhalten. Gemäß der Markownikoff-Regel entsteht dabei aus Olefinen, die an ihrer Doppelbindung mindestens drei Alkylsubstituenten tragen, ein tertiäres Thiol und aus linearen Olefinen ein sekundäres Thiol.

Sekundäre Thiole finden Anwendung als Duftstoffe, als Komponenten in Schmiermittel-Zubereitungen und als Härter für Epoxid-Harze. Des Weiteren werden sie vorteilhaft als Zwischenprodukte bei der Herstellung von oberflächenaktiven Verbindungen verwendet.

Tertiäre Thiole werden als Molmassenregulator bei Polymerisationen verwendet, insbesondere für radikalische Polymerisationen vinylischer Monomere wie beispielsweise Polymerisation von Butadien, Styrol, carboxyliertem Styrol, Acrylsäure, Acrylnitril, Acrylester, Vinylether oder ihren Gemischen.

Ullmann's Encyclopedia of Industrial Chemistry, 6th edition, 2000 Electronic Release (Wiley VCH Verlag GmbH, Weinheim, Deutschland, 2000) gibt unter dem Stichwort "Thiols and Organic Sulfides", Punkt 1.3., "Production of Aliphatic Thiols", unter Punkt 1.3.2. "From Alkenes" einen Überblick über bekannte Methoden zur Herstellung von Alkylmercaptanen. Gängige Olefingemische, die mit Schwefelwasserstoff an einem sauren Katalysator zu tert.-Dodecylmercaptan umgesetzt werden, sind trimerisiertes iso-Buten und tetramerisiertes Propen. Beides sind bekannte Gemische stark verzweigter Alkene, die früher in relativ großem Umfang zur Herstellung von anionischen Tensiden eingesetzt wurden. Bei der Wahl des Katalysators ist darauf zu achten, dass das eingesetzte Olefin oder Olefingemisch am gewählten Katalysator keine zu hohe Polymerisationsneigung aufweist, da dieser, falls sich Oligomere oder Polymere auf ihm ansammeln, deaktiviert wird, was einen häufigeren Katalysatorwechsel nötig macht und dadurch die Wirtschaftlichkeit des Verfahrens beeinträchtigen kann.

P. Bapseres, Chim. Ind. (Paris) 90 (1963), S. 358 ff., offenbart ein Verfahren zur Herstellung von tert.-Dodecylthiol aus tetramerem Propen und Schwefelwasserstoff bei -40 °C in Gegenwart von Bortrifluorid oder Aluminiumtrichlorid als Katalysator. J. F. Franz und K. I. Glass, Chem. Eng. Prog. 59 (Heft 7), 1963, Seite 68 ff., lehren ein Verfahren zur Herstellung von tert.-Dodecylthiol aus tetramerem Propen und Schwefelwasserstoff bei 49 °C bis 71 °C in Gegenwart von Bortrifluorid als Katalysator.

EP 0122654 offenbart ein Verfahren zur Herstellung von sekundären Thiolen mit 10 bis 22 Kohlenstoffatomen bei einer Temperatur von 40 bis 140°C und einem Druck von 10 bis 100 bar in Gegenwart eines Zeoliths als Katalysator.

GB 625 646 beschreibt ein Verfahren zur Schwefelwasserstoffaddition an trimeres iso-Buten mit Ton als Katalysator, der wahlweise mit Schwefel- oder Phosphorsäure aktiviert wird.

US 3,214,386 lehrt die Verwendung einer Mischung aus Phosphorsäure, Bortrifluorid und einem Alkohol mit 1 bis 5 Kohlenstoffatomen als Katalysator bei der Addition von Schwefelwasserstoff an Olefine mit 9 bis 16 Kohlenstoffatomen.

Bei den aus dem Stand der Technik bekannten Verfahren werden Feststoffsäuren wie Ionenaustauscherharze (IOT) und Zeolithe oder Gemische von Phosphorsäure, Bortrifluorid und einem Alkohol als Katalysatoren bei der Addition von Schwefelwasserstoff an Doppelbindungen eingesetzt.

Nachteile des Einsatzes von festen Verbindungen als Katalysator sind:

- Transportvorgänge innerhalb des Feststoffs (Pore) stellen oftmals den geschwindigkeitsbestimmenden Reaktionsschritt dar. Große Reaktoren mit großen Katalysatormengen sind die Folge.

- Ionenaustauscherharze sind temperaturempfindlich und verlangen aufgrund der exothermen Reaktion nach einer steten Wärmeabfuhr von den reaktiven Zentren. Daraus folgt teure Parallelbauweise, beispielsweise als Rohrbündelreaktoren.

- Ionenaustauscherharze sind mechanisch empfindlich und können ohne nennenswerten Abrieb nur in Festbetten eingesetzt werden.

- Zeolithe verlieren sehr schnell ihre saure Wirkung und müssen aufwendig außerhalb des Reaktors, beispielsweise bei 500 °C, regeneriert werden.

- Zeolithpulver bestehen zum Teil aus sehr kleinen Partikeln im Bereich weniger Mikrometer, die sehr aufwendig aus der Reaktionsmischung abgetrennt werden müssen. Findet die Abtrennung außerhalb des Reaktors statt, so muss außerdem der Katalysator zurückgeführt werden.

- Festbetten aus Formkörpern auf Basis Zeolithpulver werden während des Durchströmens mit z.B. Flüssigkeiten mechanisch zerrieben (Nachteil kleiner Partikel siehe oben)

Die Aufgabe der vorliegenden Erfindung ist die Bereitstellung eines Verfahrens zur Herstellung von Alkylthiolen durch Addition von Schwefelwasserstoff an Doppelbindungen von Olefinen, bei dem die oben genannten Nachteile, die durch Einsatz eines festen, sauren Katalysators entstehen, vermieden werden können.

Diese Aufgabe wird gelöst durch ein Verfahren zur Herstellung von Alkyl-Thiolen durch Addition von Schwefelwasserstoff an Doppelbindungen von $C_6$-$C_{20}$-Olefinen bei einer Temperatur von 20 bis 150 °C und einem Druck von 1 bis 40 bar, wobei die Reaktion in Gegenwart mindestens einer organischen, flüssigen Säure durchgeführt wird.

In dem Verfahren gemäß der vorliegenden Erfindung werden $C_6$-$C_{20}$-Olefine, bevorzugt $C_6$-$C_{18}$-Olefine, besonders bevorzugt $C_{10}$-$C_{16}$-Olefine, ganz besonders bevorzugt $C_{12}$-Olefine eingesetzt. Es können Mischungen von Olefinen mit unterschiedlicher Kohlenstoffanzahl und/oder unterschiedlichem Substitutionsmuster oder einheitliche Olefine eingesetzt werden. Diese Olefine bzw. Mischungen von Olefinen können beispielsweise durch Cracken von Paraffinwachs, Oligomerisierung von Ethen oder Metathese von Hexenen erhalten werden. Die resultierenden Produkte weisen größtenteils eine lineare Struktur auf, während Olefine, die durch Oligomerisierung von Propen und/oder Butenen erhalten werden, verzweigt sind.

Die erfindungsgemäß einsetzbaren Olefine können pro Molekül eine oder mehrere Doppelbindung(en) aufweisen. Bevorzugt werden Olefine eingesetzt, die pro Molekül eine Doppelbindung aufweisen, so genannte Mono-Olefine.

Die in dem erfindungsgemäßen Verfahren einsetzbaren Olefine können zum einen $\alpha$-Olefine mit einer endständigen Doppelbindung sein, zum anderen kann die Doppelbindung auch intern in dem Kohlenwasserstoff vorliegen.

Solche linearen internen Olefine können beispielsweise durch Chlorierung - Dechlorierung von Paraffinen, durch Paraffin-Dehydrierung und durch $\alpha$-Olefin-Isomerisierung erhalten werden. Bedingt durch das Herstellungsverfahren können die einsetzbaren Olefine bzw. Olefingemische Verunreinigungen, beispielsweise aromatische Verbindungen und/oder gesättigte Kohlenwasserstoffe, zu einem Anteil von bis zu 3 Gew.-% enthalten. Diese Verunreinigungen beeinflussen das erfindungsgemäße Verfahren nicht.

Die Olefine können linear sein oder ein oder mehrere Alkylsubstituenten entlang der Kohlenstoffhauptkette aufweisen. Werden in dem erfindungsgemäßen Verfahren Olefine eingesetzt, deren Doppelbindung endständig ist oder zwei Substituenten aufweist, so werden sekundäre Thiole erhalten, d.h., dass das die -SH-Funktionalität tragende Kohlenstoffatom mit zwei weiteren Kohlenstoffatomen verbunden ist. Werden Olefine eingesetzt, die an der Doppelbindung wenigstens drei Substituenten tragen, so werden tertiäre Thiole erhalten, d.h., dass das die -SH-Funktionalität tragende Kohlenstoffatom mit drei weiteren Kohlenstoffatomen verbunden ist. Durch das erfindungsgemäße Verfahren werden bevorzugt tertiäre Alkylthiole hergestellt.

Ganz besonders bevorzugt werden in dem erfindungsgemäßen Verfahren Olefine mit 12 Kohlenstoffatomen eingesetzt. Durch das Herstellungsverfahren bedingt kann die eingesetzte Olefinkomponente Verunreinigungen durch Olefine mit einer von 12 abweichenden Kohlenstoffanzahl bis zu einem Anteil von 5 Gew.-%, bevorzugt bis zu einem Anteil von 3 Gew.-% aufweisen.

Die in dem erfindungsgemäßen Verfahren einsetzbaren Dodecene entsprechen insbesondere bevorzugt einem oder mehreren Olefinen, die von den folgenden Verbindungen abgeleitet sind.

Olefine, die abgleitet sind von n-Dodecan (I)

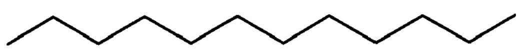

I ,

Olefine, die abgleitet sind von 5-Methyl-n-undecan (II)

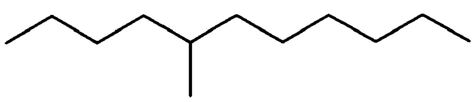

II ,

Olefine, die abgeleitet sind von 4-Ethyl-n-decan (III)

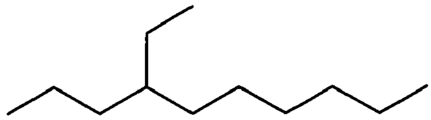

III ,

Olefine, die abgeleitet sind von 5,6-di-Methyl-n-decan (IV)

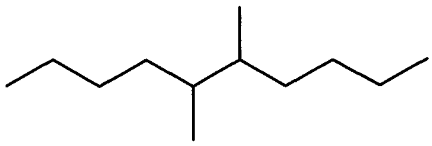

IV ,

Olefine, die abgeleitet sind von 5-Ethyl-6-methyl-n-nonan (V)

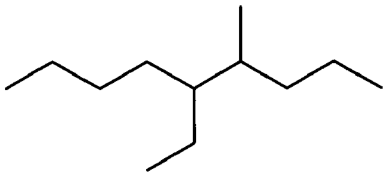

V

und Olefine, die abgleitet sind von 4,5-di-Ethyl-n-octan (VI)

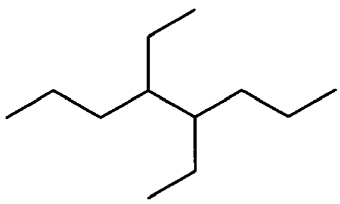

VI .

Unter "abgeleitetem Olefin" ist ein Olefin zu verstehen, das von dem betreffenden Alkan formal durch Dehydrierung, also Entfernung zweier Wasserstoffatome von benachbarten Kohlenstoffatomen unter Ausbildung einer Doppelbindung zwischen diesen Kohlenstoffatomen entsteht, wobei jedoch das Kohlenstoffgerüst unverändert bleibt. Es ist weder möglich noch notwendig, die Lage der Doppelbindung genau anzugeben, da sowohl bei üblichen Methoden der Herstellung solcher Gemische (wie beispielsweise unten angegeben) als auch bei der Umsetzung von Olefinen mit Schwefelwasserstoff die Doppelbindung entlang der Kohlenstoffkette wandert. Die Addition von Schwefelwasserstoff an Olefine in Gegenwart von sauren Katalysatoren ist eine reversible Reaktion, wobei jedoch die Doppelbindung sich anders ausbilden kann als sie vorher in der Kohlenstoffkette lag. Insgesamt stellt sich die Lage der Doppelbindung in den Kohlenstoffgerüsten und damit auch die Position der Thiolgruppe gemäß den angewendeten Bedingungen thermodynamisch oder auch kinetisch kontrolliert ein.

Bevorzugt wird in dem erfindungsgemäßen Verfahren ein Kohlenwasserstoffgemisch eingesetzt, welches mindestens 10 Gew.-%, vorzugsweise mindestens 12 Gew.-% und in besonders bevorzugter Weise mindestens 13 Gew.-%, sowie höchstens 18 Gew.-%, vorzugsweise höchstens 16 Gew.-% und in besonders bevorzugter Weise höchstens 15 Gew.-% von n-Dodecan (I) abgeleitetes Olefin,
mindestens 25 Gew.-%, vorzugsweise mindestens 30 Gew.-% und in besonders bevorzugter Weise mindestens 34 Gew.-%, sowie höchstens 40 Gew.-%, vorzugsweise höchstens 38 Gew.-% und in besonders bevorzugter Weise höchstens 36 Gew.-% von 5-Methyl-n-undecan (II) abgeleitetes Olefin,
mindestens 25 Gew.-%, vorzugsweise mindestens 30 Gew.-% und in besonders bevorzugter Weise mindestens 32 Gew.-%, sowie höchstens 40 Gew.-%, vorzugsweise höchstens 38 Gew.-% und in besonders bevorzugter Wiese höchstens 34 Gew.-% von 4-Ethyl-n-decan (III) abgeleitetes Olefin,
mindestens 2 Gew.-%, vorzugsweise mindestens 4 Gew.-% und in besonders bevorzugter Weise mindestens 5 Gew.-%, sowie höchstens 8 Gew.-%, vorzugsweise höchstens 7 Gew.-% von 5,6-di-Methyl-n-decan (IV) abgeleitetes Olefin,
mindestens 5 Gew.-%, vorzugsweise mindestens 6 Gew.-% und in besonders bevorzugter Weise mindestens 8 Gew.-%, sowie höchstens 12 Gew.-%, vorzugsweise höchstens 10 Gew.-% von 5-Ethyl-6-methyl-n-nonan (V) abgeleitetes Olefin,
mindestens 1 Gew.-%, vorzugsweise mindestens 2 Gew.-%, sowie höchstens 5 Gew.-%, vorzugsweise höchstens 4 Gew.-% und in besonders bevorzugter Weise höchstens 3.5 Gew.-% von 4,5-Di-ethyl-n-octan (VI) abgeleitetes Olefin und höchstens 5 Gew.-%, vorzugsweise höchstens 3 Gew.-% anderer Kohlenwasserstoffe, mit der Maßgabe, dass die Summe der Anteile der Komponenten 100 Gew.-% beträgt, enthält.

Durch das erfindungsgemäße Verfahren wird bevorzugt aus Dodecen und Schwefelwasserstoff tert.-Dodecylthiol hergestellt. Dabei können neben tertiären Dodecylthiolen auch prim. und/oder sekundäre Dodecylthiole vorliegen.

Die Konfiguration (cis- oder trans-Konfigurationsisomerie) der eingesetzten Olefine ist nicht erheblich. Im Allgemeinen werden die Olefine in der Konfiguration (oder in Form des Gemisches von Konfigurationsisomeren) verwendet, in der sie erzeugt werden, was meistens der thermodynamisch vorgegebenen relativen Stabilität der Isomeren entspricht.

Die Umsetzung von Olefinen (VII) mit Schwefelwasserstoff verläuft im Allgemeinen nach dem folgenden Reaktionsschema

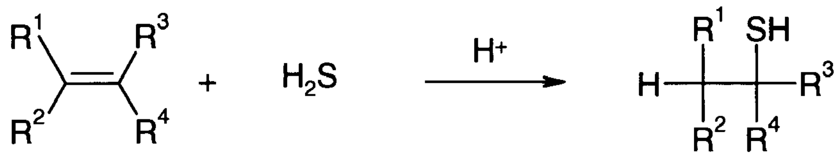


VII VIII

Die während der Reaktion gebildeten Alkyl-Thiole (VIII) können mit einem weiteren Äquivalent Olefin (VII) zu den entsprechenden Thioethern (IX) weiterreagieren

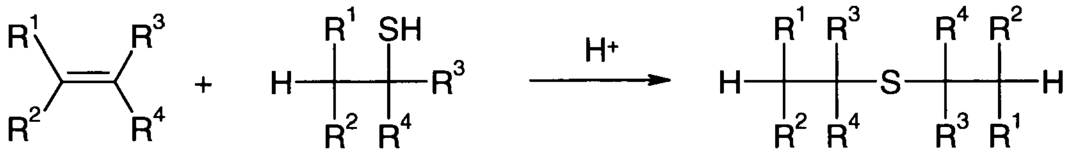


VII VIII IX

Ein Vorteil des erfindungsgemäßen Verfahrens gegenüber den Verfahren des Standes der Technik, ist, dass selektiv aus Olefinen und Schwefelwasserstoff die entsprechenden Alkylthiole (VIII) gebildet werden, ohne dass die entsprechenden Thioether (IX) in größeren Mengen entstehen.

Das erfindungsgemäße Verfahren wird bei einer Temperatur von 20 bis 150 °C, bevorzugt 30 bis 100 °C, besonders bevorzugt bei 35 bis 90 °C durchgeführt.

Das erfindungsgemäße Verfahren wird bei einem Druck durchgeführt, der über Normaldruck liegt. Dieser Druck wird durch Aufpressen von Schwefelwasserstoff, inerten Gasen wie beispielsweise Stickstoff oder Edelgasen oder Gemischen von Schwefelwasserstoff und inerten Gasen erzeugt. Nach dem Aufpressen der genannten Gase wird das

Verfahren bei einem Druck von 1 bis 40 bar, bevorzugt 5 bis 20 bar, besonders bevorzugt 7 bis 15 bar durchgeführt.

Das erfindungsgemäße Verfahren unterscheidet sich von den im Stand der Technik genannten Verfahren dadurch, dass die Umsetzung der Olefine mit Schwefelwasserstoff in Gegenwart mindestens einer organischen, flüssigen Säure durchgeführt wird.

Wesentliche Vorteile des Einsatzes von organischen, flüssigen Säuren gegenüber Feststoffsäuren sind:

- Aufgrund der höheren Säurestärke und Säurezentrendichte sind deutlich schnellere Reaktionsgeschwindigkeiten zu beobachten, daraus folgt ein entsprechend kleineres Reaktionsvolumen. Aus dem kleineren Reaktionsvolumen resultiert eine kleinere Menge der sehr giftigen Substanz Schwefelwasserstoff im Reaktor im Vergleich zu Verfahren gemäß dem Stand der Technik.

- Der Wärmetransport in bzw. aus der Reaktionsmischung bereitet aufgrund der Mischbarkeit der beteiligten Flüssigkeiten beispielsweise in Mischpumpen und Düsen keine Probleme. Feststoffe sind demgegenüber mechanisch empfindlich, werden zerrieben und in Form feinster Feststoffpartikel aus dem Reaktor ausgetragen.

Das erfindungsgemäße Verfahren kann in Gegenwart jeder organischen, flüssigen Säure durchgeführt werden. Bevorzugt werden als organische, flüssige Säuren aliphatische Carbonsäuren mit insgesamt 1 bis 12 Kohlenstoffatomen eingesetzt. Der Kohlenstoffrest kann linear oder verzweigt, gesättigt oder ungesättigt sein. Die in dem erfindungsgemäßen Verfahren einsetzbaren Carbonsäuren weisen eine bis vier, bevorzugt eine oder zwei Carbonsäurefunktionen auf.

In dem erfindungsgemäßen Verfahren können auch aromatische Carbonsäuren mit insgesamt 7 bis 15 Kohlenstoffatomen eingesetzt werden. Diese weisen eine bis vier, bevorzugt eine oder zwei Carbonsäurefunktionen auf.

In einer weiteren besonders bevorzugten Ausführungsform werden in dem erfindungsgemäßen Verfahren Alkylsulfonsäuren der allgemeinen Formel (X)

$$R\text{-}SO_3H \qquad (X)$$

eingesetzt, worin R linearer oder verzweigter, gesättigter oder ungesättigter Alkylrest mit 1 bis 10 Kohlenstoffatomen, bevorzugt linearer oder verzweigter, gesättigter Alkylrest mit 1 bis 5 Kohlenstoffatomen bedeutet. Dieser Alkylrest kann auch mit Substituenten, beispielsweise Fluoratomen, einfach, zweifach oder dreifach substituiert sein. Besonders bevorzugt wird in dem erfindungsgemäßen Verfahren eine Säure ausgewählt aus der Gruppe bestehend aus Methansulfonsäure, Trifluormethansulfonsäure, Ethansulfonsäure und Propansulfonsäure eingesetzt. Ganz besonders bevorzugt wird Methansulfonsäure eingesetzt.

Bei Einsatz von organischen, flüssigen Säuren als Katalysatoren bei der Addition von Schwefelwasserstoff an die Doppelbindung(en) von Olefinen werden hervorragende Selektivitäten und hohe Reaktionsgeschwindigkeiten erzielt.

In dem erfindungsgemäßen Verfahren werden die genannten geeigneten Säuren vorzugsweise unverdünnt eingesetzt.

Dem Reaktionsgemisch wird bevorzugt kein Lösungsmittel zugesetzt. Das Verfahren wird bevorzugt in Abwesenheit eines Lösungsmittels, in Substanz, durchgeführt.

Die organische, flüssige Säure ist bevorzugt in der Reaktionsmischung unlöslich, so dass zwei flüssige Phasen vorliegen.

Bevorzugt wird das erfindungsgemäße Verfahren durchgeführt, indem ein oder mehrere geeignete Olefine mit der flüssigen Säure zusammen in ein Reaktionsgefäß gegeben und vermischt werden. Geeignete Reaktionsgefäße sind dem Fachmann bekannt. Besonders geeignet als Reaktionsgefäß können Reaktionsmischpumpen eingesetzt werden, die eine rotationssymmetrische Mischkammer und einen darin drehangetriebenen Mischrotor aufweisen. Zur Erzielung einer besonders guten Durchmischung der mindestens zwei Komponenten ist für jede Komponente mindestens eine Einlassöffnung vorgesehen. Eine solche Mischungspumpe wird z.B. in DE 422 0239 offenbart.

In dem erfindungsgemäßen Verfahren beträgt das molare Verhältnis von Säure zu Olefin (n(Säure/n(Olefin)) bevorzugt 0,1 bis 10, besonders bevorzugt 0,5 bis 5, ganz besonders bevorzugt 0,8 bis 3.

Das erfindungsgemäße Verfahren wird bevorzugt in einem vollständig inertisierten Reaktor durchgeführt. Dazu wird beispielsweise vor Zugabe der Substrate die Luft enthaltende Gasphase, die sich in dem Reaktor befindet, durch ein inertes Gas ausgetauscht. Dieser Austausch kann durch mehrmaliges, beispielsweise zwei- oder dreimaliges, Absenken des Drucks (Evakuieren) im Reaktionsgefäß und Aufpressen des inerten Gases erfolgen. Ebenso kann der Gasaustausch durch mehrmaliges, beispielsweise zwei- oder dreimaliges, Aufpressen eines inerten Gases mit anschließendem Entspannen auf Normaldruck erfolgen. Als inertes Gas kann beispielsweise eines ausgewählt aus der Gruppe Stickstoff, Helium, Neon, Argon und Mischungen zweier oder mehrerer davon eingesetzt werden.

Die Reaktion wird durchgeführt bis der Umsatz im Allgemeinen größer 70%, bevorzugt größer 80%, besonders bevorzugt größer 85% beträgt. Das Produkt kann nach dem Fachmann bekannten Methoden isoliert werden, beispielsweise durch Phasentrennung und/oder Extraktion und gegebenenfalls gereinigt werden, beispielsweise durch Destillation. Gegebenenfalls nach der Reaktion in der Produktlösung vorliegende Säure kann durch Zugabe der entsprechenden Menge einer oder mehrerer Basen neutralisiert werden. Geeignete Basen sind dem Fachmann bekannt, genannt seien beispielsweise Kalium- und/oder Natriumhydroxid in fester oder gelöster Form.

Das erfindungsgemäße Verfahren kann kontinuierlich, semikontinuierlich oder diskontinuierlich durchgeführt werden. In einer Ausführungsform kann das erfindungsgemäße Verfahren kontinuierlich betrieben werden, d.h., dass das Produkt kontinuierlich abgeführt wird, und die Substrate gemäß ihrem Verbrauch kontinuierlich zugeführt werden, so dass im Mittel im Reaktionsgefäß konstante Konzentrationen aller vorhandenen Stoffe vorliegen. Für die kontinuierliche Verfahrensweise geeignete Reaktionsgefäße sind dem Fachmann bekannt. Beispielhaft sind Rohrreaktoren, Rührreaktoren, Umlaufreaktoren, bevorzugt jeweils mit hohem Mischenergieeintrag zum Vermischen der beiden flüssigen Phasen, genannt. Besonders geeignet als Reaktionsgefäß können Reaktionsmischpumpen eingesetzt werden, wie sie beispielsweise in DE 422 0239 offenbart werden.

In einer weiteren Ausführungsform kann das erfindungsgemäße Verfahren semikontinuierlich durchgeführt werden, d.h. dass die Substrate gemischt werden, die Reaktion gestartet wird, und entstehende Produkte kontinuierlich, beispielsweise durch Destillation, abgeführt werden.

Bei der diskontinuierlichen Verfahrensweise werden die beteiligten Substrate gemischt, die Reaktion wird gestartet, und nach Beendigung der Reaktion wird die Reaktionsmischung als Ganzes durch geeignete Methoden, beispielsweise Destillation, aufgearbeitet.

Die vorliegende Erfindung betrifft auch die Verwendung von organischen, flüssigen Säuren als Katalysator zur Erhöhung der Selektivität und/oder der Reaktionsgeschwindigkeit bei der Addition von Schwefelwasserstoff an Doppelbindungen von $C_6$-$C_{20}$-Olefinen, bevorzugt von Dodecen, zur Herstellung von Alkyl-Thiolen, bevorzugt von tert.-Dodecylthiol.

Geeignete organische Säuren wurden bereits vorstehend genannt. Besonders bevorzugt werden Alkansulfonsäuren der allgemeinen Formel (X)

$$R\text{-}SO_3H \qquad (X)$$

eingesetzt, worin R linearer oder verzweigter, gesättigter oder ungesättigter Alkylrest mit 1 bis 10 Kohlenstoffatomen, bevorzugt linearer oder verzweigter, gesättigter Alkylrest mit 1 bis 5 Kohlenstoffatomen bedeutet, der gegebenenfalls mit Substituenten, beispielsweise Fluoratomen, einfach, zweifach oder dreifach substituiert sein kann. Besonders bevorzugt ist Methansulfonsäure.

Die folgenden Beispiele erläutern die Erfindung näher, ohne sie einzuschränken.

**Beispiele**

Beispiel 1: Erfindungsgemäßes Verfahren

Es werden 10,6 g Methansulfonsäure und 40,3 g Dodecen über einen Trichter in die Anlage eingefüllt. Nach Verschließen der Anlage wird die Reaktionsmischpumpe in Betrieb genommen (Drehzahl: 2800 $min^{-1}$) und der gesamte Umpumpkreislauf, der inklusive Pumpenkopf in Doppelmantelausführung gefertigt ist, auf Starttemperatur von 40 °C gebracht. Gestartet wird die Reaktion durch Aufpressen von Schwefelwasserstoffgas auf einen Gesamtdruck von 14 bar. Der Reaktionsfortschritt wird als Funktion der Reaktionszeit gemessen (siehe Abbildung 1). Hierzu entnimmt man dem System mittels Dreiwegehahn Proben. Es wurde darauf geachtet, dass der Flüssigkeits-Füllstand der Anlage nicht so weit absinkt, dass die Reaktionsmischpumpe trocken läuft, d.h., dass die Flüssigkeitsmenge oberhalb der minimalen Füllstandshöhe zu Beginn des Experiments mindestens der Summe aller gezogenen Probenvolumina entsprechen muss. Durch die Druckabsenkung am Probenentnahmehahn und anschließendem Durchleiten von Stickstoff durch die entnommene Flüssigprobe entweicht der gelöste Schwefelwasserstoff. Anschließend wird die in der organischen Phase gelöste Methansulfonsäure (w = 0,2 %) mit Natronlauge neutralisiert. Die von Säure befreite organische Flüssigphase wird mittels Gaschromatographie analysiert, wobei zwischen Edukt (Olefin), Hauptprodukt (Thiol) und Nebenkomponente (Thioether) unterschieden werden kann. Die Ergebnisse sind in Tabelle 1 aufgeführt.

**Tabelle 1:**

| t/min | Umsatz / % | Selektivität / % | Ausbeute / % |
|---|---|---|---|
| 0 | 3,81 | 27,81 | 1,06 |
| 1 | 27,98 | 89,57 | 25,06 |
| 2 | 55,53 | 94,73 | 52,61 |
| 3 | 58,02 | 95,47 | 55,39 |

Tabelle fortgesetzt

| t/min | Umsatz / % | Selektivität / % | Ausbeute / % |
|---|---|---|---|
| 5 | 59,78 | 95,98 | 57,38 |
| 10 | 68,04 | 96,33 | 65,54 |
| 30 | 81,78 | 97,20 | 79,49 |
| 240 | 89,56 | 97,75 | 87,55 |

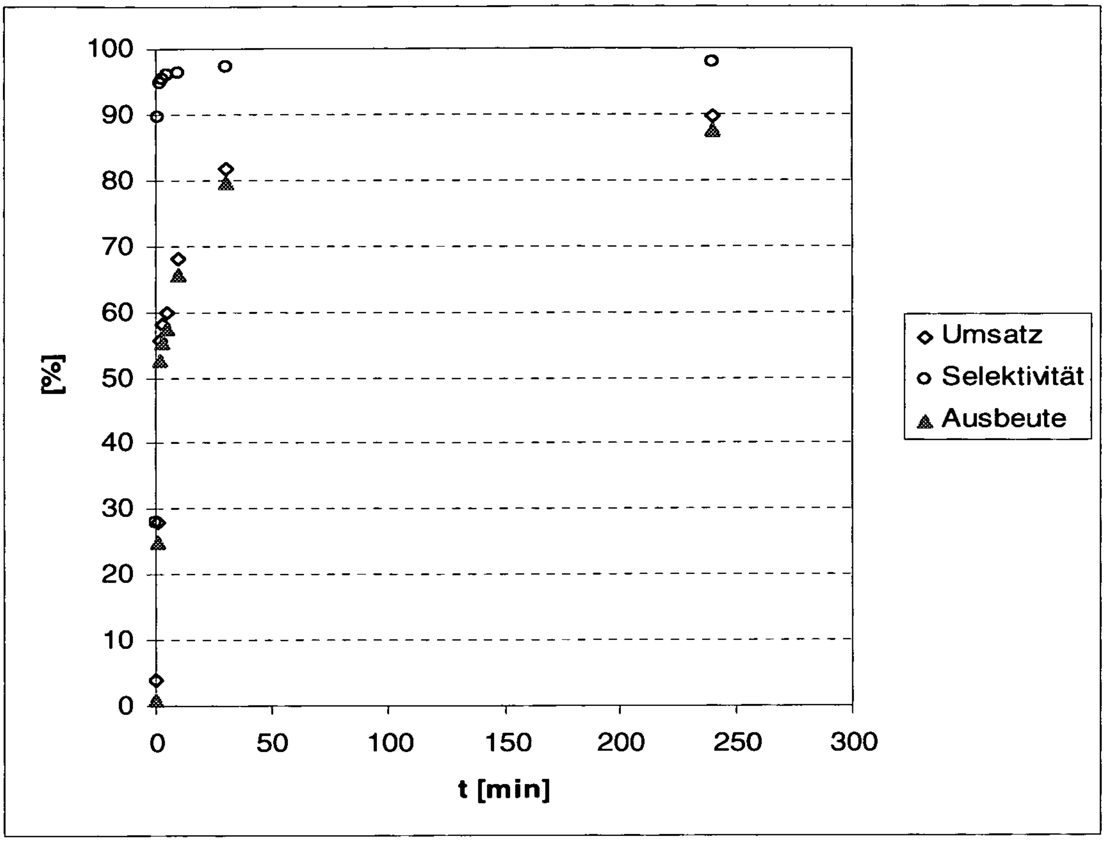


**Abbildung 1**

Beispiel 2: Diskontinuierliche Rührbehälterexperimente zum Vergleich von Methansulfonsäure mit Schwefelsäure

Die Durchführung von diskontinuierlichen Experimenten zum Testen verschiedener Flüssigsäurekatalysatoren wird in einem gerührten Autoklaven (V = 0,3 1) durchgeführt, der mit einem Begasungsrührer, Strombrechern und einer temperierbaren Doppelmantelhülle ausgestattet ist.

Dodecen und Flüssigsäure werden vor dem Verschließen des Reaktors eingefüllt. Anschließend temperiert man den Reaktorinhalt bei laufendem Rührmotor auf die gewünschte Reaktionstemperatur und verdrängt die Luft enthaltende Gasphase des Reaktors durch mehrmaliges Aufpressen von 10 bar Stickstoff und jeweiligem Entspannen auf Normaldruck. Vor dem Einleiten des Schwefelwasserstoffs wird der Rührer abgeschaltet, so dass während des dreimaligen Aufpressens von $H_2S$ (g) mit anschließendem Entspannen möglichst wenig Schwefelwasserstoff in Lösung geht. Nun wird endgültig Schwefelwasserstoff auf den gewünschten Reaktionsdruck in das System eingepresst und unmittelbar darauf der Rührer des Autoklaven gestartet. Während des Experiments bleibt die $H_2S$-Zuleitung des Reaktors geöffnet, so dass die abreagierte Schwefelwasserstoffmenge in das Reaktionssystem nachgeliefert wird. Die quantitative wie qualitative Auswertung dieser Experimente erfolgt durch den zeitlichen $H_2S$-Verbrauch und gaschromatographischer Analysen der Flüssigphase.

Die Versuche wurden jeweils mit 60 g Dodecen, bei einer Temperatur von 80 °C, einem $H_2S$-Druck von 10 bar und einer Drehzahl des Rührers von 1600 $min^{-1}$ durchgeführt. Die Ergebnisse sind in Tabelle 2 aufgeführt:

Tabelle 2

| Versuch | Säure | m(Säure) [g] | $\frac{n(Säure)}{n(Olefin)}$ | Zeit [min] | GC-Analysen-Ergebnisse [Gew.-%] | | |
|---|---|---|---|---|---|---|---|
| | | | | | Dodecen | Thiol | Thioether |
| 1 | $H_2SO_4$ | 93,8 | 2,69 | 45 | 45,1 | 3,2 | 51,7 |
| 2 | $CH_3$-$SO_3H$ | 40,0 | 1,17 | 36 | 2,5 | 91,7 | 5,8 |

Die Ergebnisse in Tabelle 2 lassen erkennen, dass mit Schwefelsäure im Vergleich zu Methansulfonsäure trotz deutlich höherer molarer Einsatzmenge keine hohe Umsatzrate erreicht wird. Darüber hinaus reagiert bei Einsatz von Schwefelsäure ein Großteil der gebildeten Thiol-Menge zu dem Folgeprodukt Thioether weiter, während die Reaktion mit Methansulfonsäure selektiv die entsprechende Thiol-Verbindung liefert.

**Patentansprüche**

**1.** Verfahren zur Herstellung von Alkyl-Thiolen durch Addition von Schwefelwasserstoff an Doppelbindungen von $C_6$-$C_{20}$-Olefinen bei einer Temperatur von 20 bis 150 °C und einem Druck von 1 bis 40 bar, **dadurch gekennzeichnet, dass** die Reaktion in Gegenwart mindestens einer organischen, flüssigen Säure durchgeführt wird.

**2.** Verfahren nach Anspruch 1, **dadurch gekennzeichnet, dass** die Reaktion bei einer Temperatur von 30 bis 100 °C durchgeführt wird.

**3.** Verfahren nach Anspruch 1 oder 2, **dadurch gekennzeichnet, dass** $C_6$-$C_{18}$-Olefine eingesetzt werden.

**4.** Verfahren nach einem der Ansprüche 1 bis 3, **dadurch gekennzeichnet, dass** tertiäre Alkylthiole hergestellt werden.

**5.** Verfahren nach Anspruch 4, **dadurch gekennzeichnet, dass** aus Dodecen und Schwefelwasserstoff tert.-Dodecylthiol hergestellt wird.

**6.** Verfahren nach einem der Ansprüche 1 bis 5, **dadurch gekennzeichnet, dass** die Säure ausgewählt ist aus der Gruppe bestehend aus organischen Carbonsäuren mit insgesamt 1 bis 12 Kohlenstoffatomen und Alkylsulfonsäuren der allgemeinen Formel (X)

$$R\text{-}SO_3H \qquad (X),$$

worin R linearer oder verzweigter, gesättigter oder ungesättigter Alkylrest mit 1 bis 10 Kohlenstoffatomen bedeutet.

**7.** Verfahren nach einem der Ansprüche 1 bis 6, **dadurch gekennzeichnet, dass** die Säure Methansulfonsäure ist.

**8.** Verwendung von organischen, flüssigen Säuren als Katalysator zur Erhöhung der Selektivität und/oder Reaktionsgeschwindigkeit bei der Addition von Schwefelwasserstoff an Doppelbindungen von $C_6$-$C_{20}$-Olefinen zur Herstellung von Alkyl-Thiolen.

**9.** Verwendung nach Anspruch 8, **dadurch gekennzeichnet, dass** die Säure Methansulfonsäure ist.

**10.** Verwendung nach Anspruch 8 oder 9, **dadurch gekennzeichnet, dass** durch Addition von Schwefelwasserstoff an Dodecen tert.-Dodecylthiol hergestellt wird.

Europäisches Patentamt

# EUROPÄISCHER RECHERCHENBERICHT

Nummer der Anmeldung
EP 05 02 7316

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | GB 684 096 A (STANDARD OIL COMPANY) 10. Dezember 1952 (1952-12-10) * Seite 1, Spalte 1, Zeile 13 - Zeile 41 * * Seite 2, Spalte 1, Zeile 4 - Zeile 15 * * Seite 2, Spalte 1, Zeile 33 - Zeile 37 * * Seite 4; Tabelle * ----- | 1-10 | C07C319/04 C07C321/04 |
| X | US 4 347 384 A (FIELDS ET AL) 31. August 1982 (1982-08-31) * das ganze Dokument * ----- | 1-6,8,10 | |
| D,A | GB 625 646 A (SHARPLES CHEMICALS INC) 1. Juli 1949 (1949-07-01) * das ganze Dokument * ----- | 1-10 | |
| A | EP 0 101 356 A (SOCIETE NATIONALE ELF AQUITAINE) 22. Februar 1984 (1984-02-22) * das ganze Dokument * ----- | 1-10 | |
| D,A | BAPSERES P: "LA SYNTHESE DES MERCAPTANS" CHIMIE ET INDUSTRIE, PARIS, FR, Bd. 90, Nr. 4, Oktober 1963 (1963-10), Seiten 358-363, XP009042138 ISSN: 0009-4358 * das ganze Dokument * ----- | 1-10 | RECHERCHIERTE SACHGEBIETE (IPC): C07C |

Der vorliegende Recherchenbericht wurde für alle Patentansprüche erstellt

| Recherchenort | Abschlußdatum der Recherche | Prüfer |
|---|---|---|
| München | 14. März 2006 | Österle, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

3
EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 05 02 7316

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-03-2006

| Im Recherchenbericht angeführtes Patentdokument | Datum der Veröffentlichung | Mitglied(er) der Patentfamilie | Datum der Veröffentlichung |
|---|---|---|---|
| GB 684096 A | 10-12-1952 | KEINE | |
| US 4347384 A | 31-08-1982 | KEINE | |
| GB 625646 A | 01-07-1949 | KEINE | |
| EP 0101356 A | 22-02-1984 | BE 897457 A1 | 03-02-1984 |
| | | BR 8304194 A | 13-03-1984 |
| | | CA 1196023 A1 | 29-10-1985 |
| | | DE 3360187 D1 | 20-06-1985 |
| | | DE 101356 T1 | 19-07-1984 |
| | | ES 8403864 A1 | 01-07-1984 |
| | | FR 2531426 A1 | 10-02-1984 |
| | | IN 158218 A1 | 27-09-1986 |
| | | IT 1165471 B | 22-04-1987 |
| | | JP 1654488 C | 13-04-1992 |
| | | JP 3016949 B | 06-03-1991 |
| | | JP 59093042 A | 29-05-1984 |
| | | NO 832803 A | 06-02-1984 |
| | | PL 243305 A1 | 30-07-1984 |
| | | SU 1220569 A3 | 23-03-1986 |
| | | US 4565893 A | 21-01-1986 |
| | | ZA 8305713 A | 25-04-1984 |

EPO FORM P0461

Für nähere Einzelheiten zu diesem Anhang : siehe Amtsblatt des Europäischen Patentamts, Nr.12/82